(19) **Europäisches Patentamt**
**European Patent Office**
**Office européen des brevets**

(11) **EP 3 636 146 B1**

(12) **EUROPEAN PATENT SPECIFICATION**

(45) Date of publication and mention
of the grant of the patent:
**09.08.2023 Bulletin 2023/32**

(21) Application number: **19202001.4**

(22) Date of filing: **08.10.2019**

(51) International Patent Classification (IPC):
***A61B 5/021*** *(2006.01)*

(52) Cooperative Patent Classification (CPC):
**A61B 5/02108;** A61B 5/02416; A61B 2560/0223

(54) **APPARATUS AND METHOD FOR ESTIMATING BLOOD PRESSURE**

VORRICHTUNG UND VERFAHREN ZUR BLUTDRUCKSCHÄTZUNG

APPAREIL ET PROCÉDÉ D'ESTIMATION DE LA PRESSION SANGUINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.10.2018 KR 20180120641**

(43) Date of publication of application:
**15.04.2020 Bulletin 2020/16**

(73) Proprietor: **Samsung Electronics Co., Ltd.
Gyeonggi-do 16677 (KR)**

(72) Inventors:
• **PARK, Chang Soon**
  **Chungju-si, Chungcheongbuk-do (KR)**
• **KWON, Ui Kun**
  **Hwaseong-si, Gyeonggi-do (KR)**
• **YOON, Seung Keun**
  **Seocho-gu, Seoul (KR)**
• **JANG, Dae Geun**
  **Yongin-si, Gyeonggi-do (KR)**

(74) Representative: **Grünecker Patent- und
Rechtsanwälte
PartG mbB
Leopoldstraße 4
80802 München (DE)**

(56) References cited:
EP-A1- 0 956 815     US-A- 5 533 511
US-A1- 2017 181 649     US-A1- 2018 177 465

**Description**

BACKGROUND

**1. Field**

Apparatuses and methods consistent with example embodiments relate to cuffless blood pressure estimation, and more particularly, an apparatus and a method for estimating blood pressure, and an apparatus for supporting blood pressure estimation.

**2. Description of Related Art**

Recently, active research has been conducted on Internet technology (IT)-medical convergence technology, which is a combination of IT technology and medical technology, due to the aging population structure, rapidly growing medical expenses, and the shortage of professional medical service personnel. The monitoring of the health status of the human body is not limited to the hospital, but is expanding to the field of mobile health care, which monitors the health status of users moving in everyday life, such as home and office. Archetypal examples of bio-signals indicating the individual's health status may include an electrocardiography (ECG) signal, a photoplethysmogram (PPG) signal, an electromyography (EMG) signal, and the like. Various bio-signal sensors are being developed to measure such signals in daily life. In the case of a PPG sensor, it is possible to estimate blood pressure of a human body by analyzing pulse waveforms that reflect a cardiovascular status.

According to research on PPG bio-signals, the entire PPG signal is a superposition of a propagation wave propagating from the heart to peripheral parts of a body and reflection waves returning from the peripheral parts of the body. It is known that by extracting various features associated with the propagation wave or the reflection waves, information from which blood pressure can be estimated can be obtained.

US 2018/177465 A1 refers to a bio-signal feature scaling apparatus that includes a processor configured to execute instructions to extract a feature from a bio-signal of an object, to estimate a cause of change in a bio-state of the object, to calibrate a scale factor based on the estimated cause of change in the bio-state, and to scale the extracted feature by applying the calibrated scale factor to the extracted feature.

EP 0 956 815 A1 refers to an apparatus for non-invasive estimation of the arterial blood pressure of a living subject. The apparatus comprises first means for obtaining the velocity of propagation of a pulse wave, second means for obtaining at least one between the heart rate and an area defined by a volume pulse wave, third means for estimating, according to a predetermined relationship between blood pressure, and pulse wave propagation velocity and at least one of heart rate and pulse wave area, the blood pressure, the predetermined relationship being defined by a numerical expression including a plurality of predetermined coefficients, and fourth means for selecting one group of coefficients which corresponds to a blood pressure of the subject.

US 2017/181649 A1 refers to systems and methods for determining a blood pressure of a subject from a photoplethysmogram. The systems and methods include acquiring raw photoplethysmogram, PPG, data from a subject by measuring light reflected from or transmitted through a portion of the subject's body, determining a mean beat shape from the raw PPG data, and analyzing the mean beat shape to determine a blood pressure of the subject. The method can include analyzing a distinct shape of the mean beat shape to determine the blood pressure. The method can include identifying individual beats within the raw PPG data, collecting motion data and filtering the individual beats against the motion data, measuring biometric features of filtered beats, scaling individual beats in time and amplitude, and measuring additional shape features of scaled beats.

US 5 533 511 A refers to a blood pressure monitor for determining a patient's blood pressure. The blood pressure monitor comprises a processor attached to a first input device for receiving an initial input representing the patient's absolute blood pressure, and a noninvasive sensor attached to the patient for measuring at least one physiological function. The processor executes a procedure for evaluating the initial input and the sensed physiological function to determine the patient's blood pressure. A method for determining a patient's blood pressure comprises the steps of storing an initial input representing a patient's absolute blood pressure, noninvasively sensing at least one of the patient's physiological functions, and evaluating the initial input and the sensed input to determine the patient's blood pressure.

SUMMARY

It is the object of the present invention to provide an improved apparatus, method, and computer-readable medium for estimating a blood pressure.

This object is solved by the subject matter of the independent claims.

Preferred embodiments are defined by the dependent claims.

In accordance with an aspect of an example embodiment, there is provided an apparatus for estimating a blood pressure, the apparatus including a sensor configured to measure a bio-signal, and a processor configured to acquire a feature value from the bio-signal, detect a blood pressure change sign, based on the feature value, acquire a scale factor, based on the blood pressure change sign, and estimate the blood pressure, based on the feature value and the scale factor.

The processor is further configured to detect the blood pressure change sign, based on whether the acquired feature value is greater than a reference feature value.

The reference feature value is acquired from a bio-signal that is measured at a time of calibration.

The processor may be further configured to detect that the blood pressure change sign is positive, based on the acquired feature value being greater than the reference feature value, and detect that the blood pressure change sign is negative, based on the acquired feature value being less than the reference feature value.

The processor may be further configured to acquire the scale factor, using a scale factor estimation model that is defined differently according to the blood pressure change sign.

The scale factor estimation model may include any one or any combination of a constant value, a first estimation equation reflecting characteristics of users, and a second estimation equation reflecting characteristics of each of groups of the users.

The processor may be further configured to acquire the scale factor as a first constant value that is defined for a positive sign, based on the blood pressure change sign being detected to be positive, and acquire the scale factor as a second constant value that is defined for a negative sign, based on the blood pressure change sign being detected to be negative.

The processor may be further configured to acquire the scale factor as the constant value that is defined for a positive sign, based on the blood pressure change sign being detected to be positive, and acquire the scale factor, using the second estimation equation that is defined for a negative sign, based on the blood pressure change sign being detected to be negative.

The processor may be further configured to, based on the blood pressure change sign being determined to be negative, receive characteristic information of one of the users, and acquire the scale factor, based on the characteristic information being applied to the second estimation equation for one of the groups to which the one of the users belongs.

Each of the groups may be classified based on any one or any combination of sex, age, whether medication is taken, occupation, and disease.

Each of the first estimation equation and the second estimation equation may have an input of a personal characteristic factor reflecting at least one of the characteristics of one of the users.

The personal characteristic factor may include any one or any combination of an age, a sex, a height, a weight, a body mass index, a pulse pressure, a baseline systolic blood pressure, a baseline diastolic blood pressure, a difference between the baseline systolic blood pressure and the baseline diastolic blood pressure, and a heart rate.

The processor may be further configured to acquire the feature value by combining one or more of a shape of a waveform of the bio-signal, a time and an amplitude at a maximum point of the bio-signal, a time and an amplitude at a minimum point of the bio-signal, a time and an amplitude at a position of a pulse waveform component constituting the bio-signal, and an area of the bio-signal.

The processor may be further configured to scale the feature value with the scale factor, and estimate the blood pressure, based on the scaled feature value.

The processor may be further configured to estimate the blood pressure, further based on a baseline blood pressure value at a time of calibration.

The sensor may include a light source configured to emit light to an object of interest, and a detector configured to detect light that is scattered from the object of interest.

The apparatus may further include an output interface configured to output a processing result of the processor.

The apparatus may further include a communication interface configured to receive a scale factor estimation model to be used to acquire the scale factor, from an external device.

The processor may be further configured to determine whether to update the scale factor estimation model, based on any one or any combination of a preset period, a change in characteristics of a user, and the estimated blood pressure, and control the communication interface to receive a new scale factor estimation model from the external device, based on the scale factor estimation model being determined to be updated.

In accordance with an aspect of an example embodiment, there is provided a method of estimating a blood pressure, the method including measuring a bio-signal, acquiring a feature value from the bio-signal, detecting a blood pressure change sign, based on the feature value, acquiring a scale factor, based on the blood pressure change sign, and estimating the blood pressure, based on the feature value and the scale factor.

The detecting of the blood pressure change sign includes detecting the blood pressure change sign, based on whether the acquired feature value is greater than a reference feature value.

The acquiring of the scale factor may include acquiring the scale factor, using a scale factor estimation model that is defined differently according to the blood pressure change sign.

The scale factor estimation model may include any one or any combination of a constant value, a first estimation equation reflecting characteristics of users, and a second estimation equation reflecting characteristics of each of groups of the users.

The acquiring of the scale factor may include acquiring the scale factor as the constant value that is defined for a positive sign, based on the blood pressure change sign being detected to be positive, and acquiring the scale factor, using the second estimation equation that is defined for a negative sign, based on the blood pressure change sign being detected to be negative.

The acquiring of the scale factor may include, based on the blood pressure change sign being determined to be negative, selecting one of the groups to which one of the users belongs, based on characteristic information of the one of the users.

The estimating of the blood pressure may include scaling the feature value with the scale factor, and estimating the blood pressure, based on the scaled feature value.

In accordance with an aspect of an example embodiment, there is provided an apparatus for supporting blood pressure estimation, the apparatus including an information collector configured to collect blood pressure-related information of users, and a processor configured to generate, based on the blood pressure-related information, a scale factor estimation model for each of a positive blood pressure change sign and a negative blood pressure change sign, the scale factor estimation model being for scaling a bio-signal feature value to be used in estimating a blood pressure.

The scale factor estimation model may include any one or any combination of a constant value, a first estimation equation reflecting characteristics of the users, and a second estimation equation reflecting characteristics of each of groups of the users.

The processor may be further configured to classify the users, based on a blood pressure change sign of a bio-signal feature value for each of the users, acquire an optimal scale factor of each of the users, based on the bio-signal feature value and an actual blood pressure value of each of the users, the bio-signal feature value and the actual blood pressure value corresponding to the blood pressure change sign of each of the users, and generate the first estimation equation reflecting the characteristics of the users, based on a personal characteristic factor and the optimal scale factor of each of the users.

The processor may be configured to classify the users, based on a blood pressure change sign of a bio-signal feature value for a respective one of the users, sub-classify the classified users into the groups, based on any one or any combination of sex, age, whether medication is taken, occupation, and disease, and generate the second estimation equation reflecting the characteristics of each of the groups.

The apparatus may further include a communication interface configured to transmit the scale factor estimation model to an apparatus for estimating blood pressure, based on a request being received from the apparatus for estimating blood pressure or based on the scale factor estimation model being generated.

The apparatus may further include a storage configured to store either one or both of the blood pressure-related information and the scale factor estimation model.

In accordance with an aspect of an example embodiment, there is provided an apparatus for estimating a blood pressure, the apparatus including a sensor configured to measure a bio-signal of a user, and a processor configured to acquire a current feature value from the bio-signal, determine whether the current feature value is greater than a reference feature value, based on the current feature value being determined to be greater than the reference feature value, acquire a scale factor, using a first model, based on the current feature value being determined to be less than the reference feature value, acquire the scale factor, using a second model different from the first model, scale the current feature value with the scale factor, and estimate the blood pressure, based on the scaled current feature value.

The processor may be further configured to, based on the current feature value being determined to be greater than the reference feature value, select a group to which the user belongs, based on characteristic information of the user, and acquire the scale factor, using an estimation equation that is preset for the selected group and for when the current feature value being determined to be greater than the reference feature value, and based on the current feature value being determined to be less than the reference feature value, setting the scale factor to a value that is preset for when the current feature value is determined to be less than the reference feature value.

The processor may be further configured to select a group to which the user belongs, based on characteristic information of the user, based on the current feature value being determined to be greater than the reference feature value, acquire the scale factor, using a first estimation equation that is preset for the selected group and for when the current feature value being determined to be greater than the reference feature value, and based on the current feature value being determined to be less than the reference feature value, acquire the scale factor, using a second estimation equation that is preset for the selected group and for when the current feature value being determined to be less than the reference feature value.

BRIEF DESCRIPTION OF THE DRAWINGS

The above and/or other aspects will be more apparent from the following description of example embodiments taken in conjunction with the accompanying drawings, in which:

FIGS. 1A and 1B are block diagrams illustrating an apparatus for estimating blood pressure according to embodiments;

FIGS. 2A and 2B are block diagrams illustrating a processor in accordance with the embodiments of FIGS. 1A and 1B;

FIGS. 3A, 3B, 3C, 3D, 3E, 3F and 3G are diagrams illustrating blood pressure estimation according to embodiments;

FIG. 4 is a flowchart illustrating a method of estimating blood pressure according to embodiments;

FIGS. 5A, 5B, 5C and 5D are flowcharts illustrating acquisition of a scale factor according to embodiments;

FIG. 6 is a block diagram illustrating an apparatus for supporting blood pressure estimation according to embodiments;

FIGS. 7A and 7B are diagrams illustrating a wearable device; and

FIG. 8 is a diagram illustrating a smart device.

DETAILED DESCRIPTION

The following detailed description is provided to assist the reader in gaining a comprehensive understanding of the methods, apparatuses and/or systems described herein. Various changes, modifications, and equivalents of the systems, apparatuses and/or methods described herein will suggest themselves to those of ordinary skill in the art. In the following description, a detailed description of known functions and configurations incorporated herein will be omitted when it may obscure the subject matter with unnecessary detail. Throughout the drawings and the detailed description, unless otherwise described, the same drawing reference numerals will be understood to refer to the same elements, features, and structures. The relative size and depiction of these elements may be exaggerated for clarity, illustration, and convenience.

As used herein, the singular forms are intended to include the plural forms as well, unless the context clearly indicates otherwise. It will be further understood that the terms "comprises" and/or "comprising," or "includes" and/or "including" when used in this description, specify the presence of stated features, numbers, steps, operations, elements, components or combinations thereof, but do not preclude the presence or addition of one or more other features, numbers, steps, operations, elements, components or combinations thereof. In addition, terms such as "unit" and "module" denote units that process at least one function or operation, and they may be implemented by using hardware, software, or a combination of hardware and software.

Hereinafter, embodiments of an apparatus and method for estimating blood pressure will be described in detail with reference to the accompanying drawings.

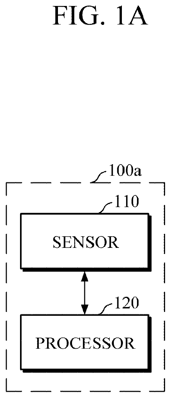

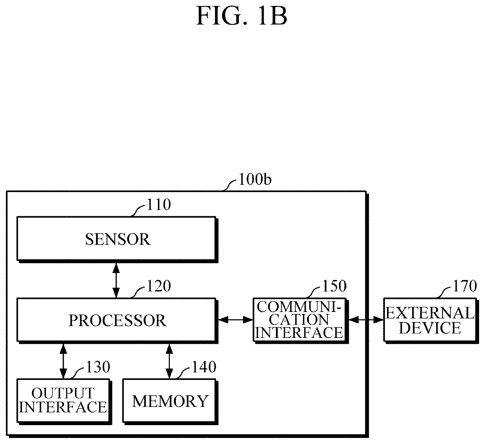

FIGS. 1A and 1B are block diagrams illustrating an apparatus for estimating blood pressure according to embodiments. In the present embodiments, the apparatuses 100a and 100b for estimating blood pressure may be mounted in a terminal, such as a smartphone, a tablet personal computer (PC), a desktop PC, a notebook PC, or the like, or mounted in a wearable device in the form that can be worn on an object of interest. In this case, the wearable device may be implemented as a wristwatch type, a bracelet type, a wrist band type, a ring type, a glasses-type, or a hair band type wearable device, but is not limited thereto. In addition, the apparatuses 100a and 100b may be mounted in a medical device manufactured for the use in bio-information measurement and analysis in a medical institution.

Referring to FIGS. 1A and 1B, each of the apparatuses 100a and 100b for estimating blood pressure includes a sensor 110 and a processor 120.

The sensor 110 may measure a bio-signal from an object of interest. In this case, the bio-signal may be a pulse wave signal including a photoplethysmogram (PPG) signal. However, the bio-signal is not limited thereto and may include various types of bio-signals, such as an electrocardiography (ECG) signal, a PPG signal, an electromyography (EMG) signal, and the like, which can be modeled as a summation of a plurality of waveform components. In this case, the object of interest may be a human body area that is in contact with or adjacent to the sensor 110 and is easy to measure a pulse wave. For example, the object of interest may include a wrist skin area adjacent to a radial artery and a human body skin area where capillaries or venous blood vessels pass. However, the object of interest is not limited to the above examples and may be a peripheral part of a human body, such as a finger, a toe, or the like, which is a region having a high density of blood vessels in the human body.

The sensor 110 may include a light source and a detector. The light source may emit light to the object of interest, and the detector may detect light scattered or reflected from the object of interest. The light source may include a light emitting diode (LED), a laser diode, and a phosphor and may be configured as one or two or more arrays. The detector may include one or more pixels and each pixel may include, but not limited to, a photodiode, a photo transistor, and an image sensor, each of which detects light and converts the light into an electrical signal.

The processor 120 may be electrically connected to the sensor 110. The processor 120 may control the sensor 110 in response to a request for estimation of blood pressure from the processor 120 and receive a bio-signal from the sensor 110. The request for estimation of blood pressure may be input by a user or may occur when a predetermined cycle is reached. The processor 120 may perform preprocessing, such as filtering for removing noise, amplification of a bio-signal, or conversion of a bio-signal into a digital signal, when an electrical bio-signal is received from the sensor 110.

The processor 120 may estimate blood pressure based on the bio-signal received from the sensor 110. For example, the processor 120 may acquire a feature value by analyzing the bio-signal, scale the acquired feature value, and estimate blood pressure on the basis of the scaled feature value. The processor 120 may scale the feature value to a different extent on the basis of a blood pressure change sign and thereby estimate blood pressure by taking into account characteristics of each individual. In this case, the blood pressure change sign may indicate whether the blood pressure at the time of measurement is increased or decreased relative to the blood pressure at the time of calibration.

The feature value acquired from the bio-signal is a value that changes because the feature value reflects the characteristics of the bio-signal of each individual at the instance of measurement. On the other hand, a scale factor for scaling the feature value is given as the same constant value to most people in a limited environment in which it is difficult to sufficiently reflect the characteristics of each individual, and thus it is limited to estimate blood pressure in consideration of the characteristics of each individual. In the present embodiments, by varying the extent to which the feature value is scaled at the time of blood pressure estimation according to the situation, it is possible to estimate accurate blood pressure that reflects the characteristics of each individual.

Referring to FIG. 1B, the apparatus 100b for estimating blood pressure may further include an output interface 130, a storage 140, and a communication interface 150.

The output interface 130 may output processing results from the sensor 110 and the processor 120. For example, the output interface 130 may visually output an estimated blood pressure value through a display module. Alternatively, the output interface 130 may output the estimated blood pressure value through a speaker module or a haptic module in a nonvisual method, such as voice, vibration, tactile sensation, or the like. The output interface 130 may divide an area of the display into two or more regions, output a bio-signal graph used in estimating blood pressure, a blood pressure estimation result, and the like to a first region, and output the blood pressure estimation history in the form of a graph or the like to a second region. In this case, when the estimated blood pressure value is out of a normal range, warning information may also be output in various ways, such as being emphasized in red color, being displayed with the normal range, being output as a voice warning message, or intensity-controlled vibration, and the like.

The storage 140 may store the processing results of the sensor 110 and the processor 120 therein. In addition, the storage 140 may store a variety of reference information for blood pressure estimation. For example, the reference information may include user characteristic information, such as user's age, age, health status, and the like. The reference information may include a reference feature value at the time of calibration, a baseline blood pressure value, a blood pressure cycle, criteria of calibration determination, a scale factor estimation model, a blood pressure estimation model, a condition for acquisition of a scale factor, criteria of scale factor estimation model update, and the like. However, the reference information is not limited to the above examples.

In this case, the storage 140 may include a storage medium, such as a memory of flash memory type, hard disk type, multimedia card micro type, or card type (e.g., SD or XD memory), random access memory (RAM), static random access memory (SRAM), read-only memory (ROM), electrically erasable programmable read-only memory (EEPROM), programmable read-only memory (PROM), magnetic memory, magnetic disk, optical disk, or the like, but is not limited thereto.

The communication interface 150 may communicate with an external device using a wired/wireless communication technology under the control of the processor 120 and transmit and receive a variety of data. For example, the communication interface 150 may transmit a blood pressure estimation result to the external device 170 and a variety of reference information for blood pressure estimation, for example, a baseline blood pressure value, a scale factor estimation model, and the like, from the external device 170. In this case, the external device may include an information processing device, such as a cuff-type blood pressure measurement device, a blood pressure estimation support device, a smartphone, a tablet PC, a desktop PC, and notebook PC.

In this case, the communication technology may include Bluetooth communication, Bluetooth low energy (BLE) communication, near field communication (NFC), a wireless local area network (WLAN) communication, ZigBee communication, infrared data association (IrDA) communication, Wi-Fi direct (WFD) communication, ultra-wideband (UWB) communication, Ant+ communication, WiFi communication, radio frequency identification (RFID) communication, 3rd generation (3G) communication, 4G communication, 5G communication, etc. However, the communication technology is not limited to the above examples.

The processor 120 may determine, on the basis of a predetermined interval, a change of a user's characteristic, and a blood pressure estimation result, whether the scale factor estimation model is to be updated. For example, the processor 120 may control the communication interface 150 by referring to the reference information of the storage 140 when a predetermined update interval is reached. In addition, the processor 120 may receive the user characteristic information from the user at the time of bio-signal measurement, and determine whether to update the scale factor estimation model by determining whether the received user characteristic has changed. For example, as described below, when there is a change in information of a group to which the user is belonging, for example, when an age group, information on whether blood pressure medication is taken, or the like is changed, the processor 120 may control the communication interface 150 to update the existing estimation equation to an estimation equation of a new group. In addition, when it is determined that the accuracy of a result of estimation of blood pressure is low, the processor 120 may control the communication interface 150 to update the scale factor estimation model.

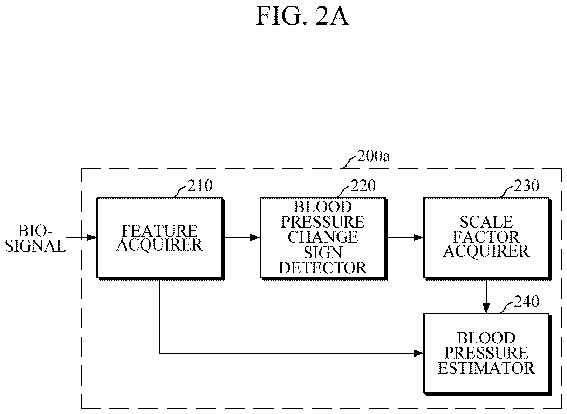

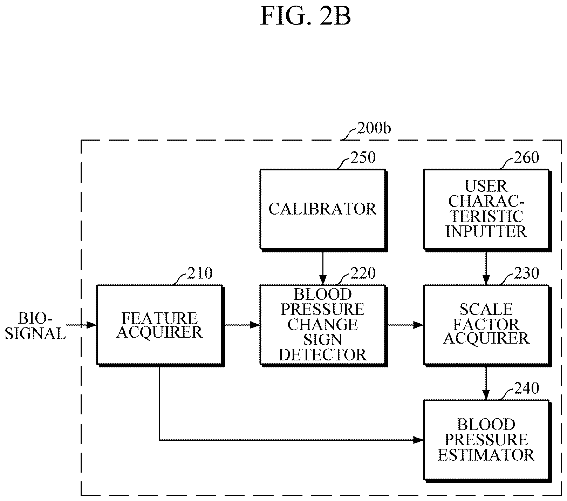

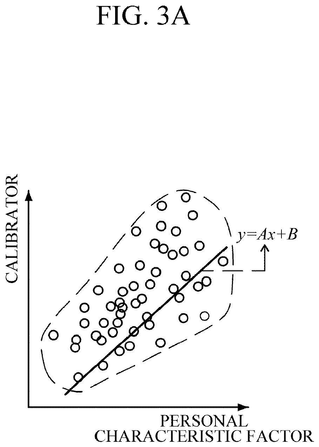

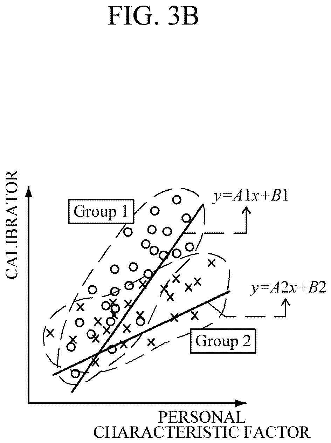

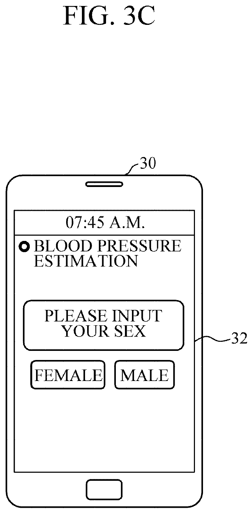

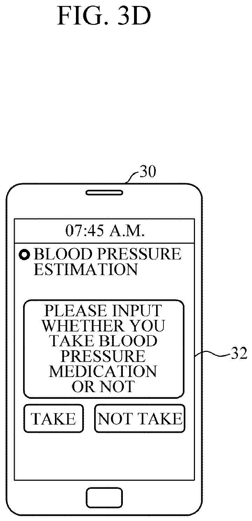

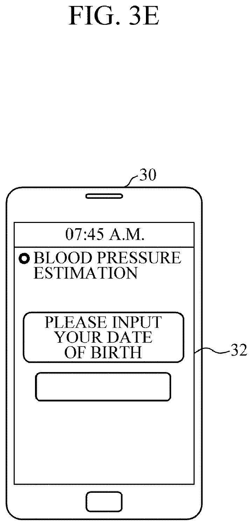

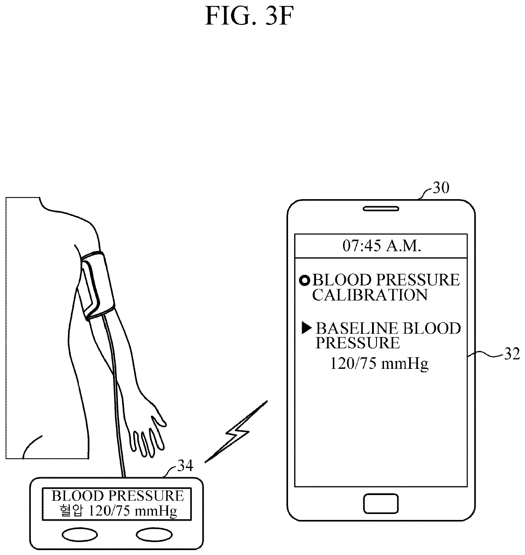

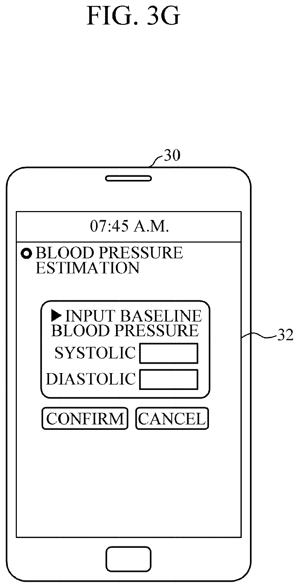

FIGS. 2A and 2B are block diagrams illustrating a processor in accordance with the embodiments of FIGS. 1A and 1B. FIGS. 3A, 3B, 3C, 3D, 3E, 3F and 3G are diagrams illustrating blood pressure estimation according to embodiments.

A pulse wave signal is a superposition of a propagation wave propagating from the heart to peripheral parts of a body and reflection waves returning from the peripheral parts. A feature highly correlated with blood pressure may be extracted by appropriately combining time and amplitude information at a position of each pulse waveform component.

Referring to FIGS. 2A and 2B, each of a processor 200a and a processor 200b may include a feature acquirer 210, a blood pressure change sign detector 220, a scale factor acquirer 230, and a blood pressure estimator 240.

The feature acquirer 210 may acquire a feature value by analyzing a bio-signal received from the sensor 110. In this case, the feature value is a value that is associated with a blood pressure change sign, and may be, for example, a value characterized in that it is increased or decreased in the same direction as the increase or decrease of the blood pressure.

For example, the feature acquirer 210 may extract, a shape of a waveform from the bio-signal, time and amplitude of a maximum point, time and amplitude at a minimum point, time and amplitude at a position of a pulse waveform component constituting the bio-signal, an area of at least one interval of the bio-signal, a heart rate, and the like, and acquire a feature value by appropriately combining the extracted pieces of information. In this case, the feature acquirer 210 may obtain a second-order derivative of the bio-signal to obtain a position of a pulse wave component constituting the bio-signal and determine a position of a minimum point of a second-order derivative signal as the position of a pulse wave component. Alternatively, the feature acquirer 210 may acquire a new feature value by appropriately combining two or more of the above-described feature values. In this case, the feature values may be combined in various ways, such as addition, subtraction, division, multiplication, log value, and a combination thereof, and is not particularly limited to any specific way.

The blood pressure change sign detector 220 may detect a blood pressure change sign on the basis of the acquired feature value. For example, the blood pressure change sign detector 220 may detect the blood pressure change sign on the basis of a direction of the acquired feature value's change relative to a reference feature value. That is, the blood pressure change sign detector 220 may determine the blood pressure change sign to be positive (+) when the acquired feature value is greater than the reference feature value, and may determine the blood pressure change sign to be negative (-) when the acquired feature value is smaller than the reference feature value. In this case, the reference feature value may be a feature value acquired from a bio-signal measured at the time of calibration.

The scale factor acquirer 230 may acquire a different scale factor according to the detected blood pressure change sign. For example, the scale factor acquirer 230 may acquire a scale factor suitable for the detected blood pressure change sign using a scale factor estimation model that is defined differently for each blood pressure change sign. In this case, the scale factor estimation model may be defined for each blood pressure change sign in advance. That is, the scale factor estimation model to be applied in the case of a positive (+) sign and the scale factor estimation model to be applied in the case of a negative (-) sign are defined differently from each other. The scale factor estimation model may include any one or any combination of a predefined constant value, a first estimation equation in which characteristics of each individual are reflected, and a second estimation equation in which characteristics of each group are reflected.

In one example, the scale factor acquirer 230 may acquire a fixed constant value predefined for the positive (+) sign as the scale factor when the detected blood pressure change sign is positive (+). In addition, when the detected blood pressure change sign is negative (-), the scale factor acquirer 230 may acquire the scale factor using the second estimation equation predefined for the negative (-) sign. When the detected blood pressure change sign is negative (-), the scale factor acquirer 230 may determine a group to which the user belongs, and may acquire a scale factor by inputting a personal feature factor of the user to the second estimation equation that is defined for the corresponding group. In this case, the personal feature factor may include the user's age, sex, height, weight, body mass index, pulse pressure, baseline systolic blood pressure, baseline diastolic blood pressure, a difference between baseline systolic blood pressure and baseline diastolic blood pressure, and a heart rate, but is not limited thereto.

In another example, when the detected blood pressure change sign is positive (+), the scale factor acquirer 230 may acquire a fixed constant value predefined for the positive (+) sign as a scale factor, and when the detected blood pressure change sign is negative (-), may acquire a fixed constant value predefined for the negative (-) sign as a scale factor. In another example, when the detected blood pressure change sign is positive (+), the scale factor acquirer 230 may acquire a scale factor using the first estimation equation predefined for the positive (+) sign when the detected blood pressure change sign is positive (+), and may acquire a scale factor using the first estimation equation predefined for the negative (-) sign when the detected blood pressure change sign is negative (-). In another example, the scale factor acquirer 230 may acquire a scale factor using the second estimation equation predefined for the positive (+) sign when the detected blood pressure change sign is positive (+), and may acquire a scale factor using the second estimation equation predefined for the negative (-) sign when the detected blood pressure change sign is negative (-).

However, the application of the scale factor estimation models for acquiring the scale factor are not limited to the above examples, and may be modified variously according to the types of devices in which the present embodiments are employed, for example, whether the device is a smartphone or a smart watch, or according to characteristics of a user who uses the device.

In addition, the first estimation equation is a function formula obtained by reflecting characteristics of each individual. For example, referring to FIG. 3A, a scatter plot for the whole of a plurality of users may be plotted, displaying a personal characteristic factor of each user on an x-axis and an optimal scale factor of each user on a y-axis and then a linear function formula, $y=Ax+B$, which reflects characteristics of the entire users may be obtained through a linear regression equation. In this case, the optical scale factor may be obtained by learning or on the basis of baseline blood pressure of each user, a feature value obtained from a bio-signal, and actual blood pressure measured at the time of bio-signal measurement, and the like. In this way, the first estimation equations for a positive (+) sign and a negative (-) sign may be obtained for the plurality of users.

In addition, the second estimation equation may be a predefined function formula by reflecting characteristics of each group. For example, referring to FIG. 3B, the plurality of users may be divided into two groups group 1 and group 2 and then an estimation equation for each group may be obtained by applying the above-described method. That is, linear function formulas, such as $y=A1x+B1$ for group 1 and $y=A2x+B2$ for group 2, may be obtained. In this way, the second estimation equations for a positive sign (+) and a negative sign (-) for each group may be obtained. In FIG. 3B, two groups are illustrated but the embodiment is not limited thereto, such that groups of users may be divided on the basis of sex, age group, whether the users take medication, occupational group, disease, or a combination thereof.

When the scale factor in accordance with the detected blood pressure change sign is acquired, the blood pressure estimator 240 may scale a feature value using the acquired scale factor and estimate blood pressure using the scaled feature value. In this case, blood pressure may be estimated by further combining the scaled feature value with an offset value. Below is Equation 1 that is an example of a blood pressure estimation equation, but the embodiment is not limited thereto.

$$BP = SF \times f_{cur} + Offset \qquad (1)$$

Here, $BP$ denotes estimated blood pressure, $SF$ denotes a scale factor obtained by the scale factor acquirer 230, and $f_{cur}$ denotes a feature value acquired by the feature acquirer 210. In this equation, $Offset$ may denote an actual baseline blood pressure value measured using a cuff blood pressure device or the like at the time of calibration, but the embodiment is not limited thereto.

Referring to FIG. 2B, the processor 200b may further include a calibrator 250 and a user characteristic inputter 260.

The calibrator 250 may perform calibration when a request for calibration is received from the user or when a preset condition for calibration is satisfied. In this case, when the preset condition for calibration is satisfied, the calibrator 250 may guide the user to perform calibration. For example, when a preset calibration interval is reached or when the total number of times that an estimated blood pressure value does not satisfy a preset normal range, the number of times that the normal range is not continuously satisfied, the number of times that the normal range is not satisfied for a predetermined period of time, or the like is greater than or equal to a predetermined threshold, it may be determined that calibration is to be performed.

When the user measures baseline blood pressure through an external blood pressure measurement device, the calibrator 250 may acquire baseline blood pressure from the external blood pressure measurement device or the user. In addition, the calibrator 250 may control the sensor 110 to measure a bio-signal for use in calibration.

In one example, referring to FIG. 3F, the calibrator 250 may control the communication interface 150 to communicate with an external blood pressure measurement device 34, and may receive a baseline blood pressure value when the external blood pressure measurement device 34 completes the measurement of baseline blood pressure. The user may measure a reference bio-signal by bringing the right finger into contact with the sensor 110 while measuring cuff blood pressure on the left arm. However, the measurements of the baseline blood pressure and the reference bio-signal are not necessarily performed simultaneously. When the baseline blood pressure value is received from the external blood pressure measurement device 34 through the communication interface 410, the calibrator 250 may output an interface to a display 32 of a device 30 in which the present embodiments are implemented, and may display the baseline blood pressure value on the output interface.

In another example, referring to FIG. 3G, the baseline blood pressure value may be directly received from the user. The calibrator 250 may output an interface through the output interface 130 to allow the user to input the baseline blood pressure value to the display 32 of the device 30.

The calibrator 250 may acquire a reference feature value from the reference bio-signal and store the acquired reference feature value, the baseline blood pressure value, and the like in the storage 140 as reference information.

The user characteristic inputter 260 may receive user characteristic information from the user to determine a group to which the user belongs for applying the above-described second estimation equation or to acquire an input value for acquiring a scale factor. In this case, the user characteristic information may include the above-described personal characteristic factor and information about whether medication including blood pressure medication that affects blood pressure is taken, health status, and the like. Only the second estimation equation for the group to which the user belongs may be stored in advance in the storage 140 and then be used. In this case, a process for receiving the user characteristic information to determine the group to which the user belongs, which will be described below, may be omitted.

Referring to FIGS. 3C to 3E, in a case in which information on a group to which the user belongs is to be determined for application of a scale factor estimation model, the user characteristic inputter 260 may output an interface through the output interface 130 and receive the user characteristic information using the interface.

For example, as shown in FIG. 3C, in a case in which the group associated with the second estimation equation is classified based on sex, an interface including an object to be used to select sex may be output, together with text, such as "please input your sex," to the display 32 of the device 30. In addition, referring to FIG. 3D, in a case in which the group associated with the second estimation equation is classified on the basis of whether blood pressure medication is taken, an interface including an object to be used to select the use of medication may be output, together with text, such as "please input whether you take blood pressure medication or not," to the display 32 of the device 30. Similarly, referring to FIG. 3E, in a case in which the group associated with the second estimation equation is classified based on age group, an interface including an object to be used to input a user's date of birth may be output, together with text, such as "please input your date of birth," to the display 32 of the device 30.

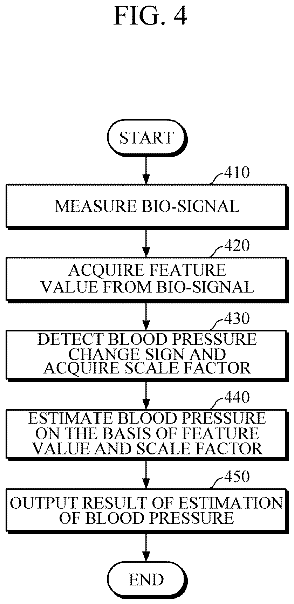

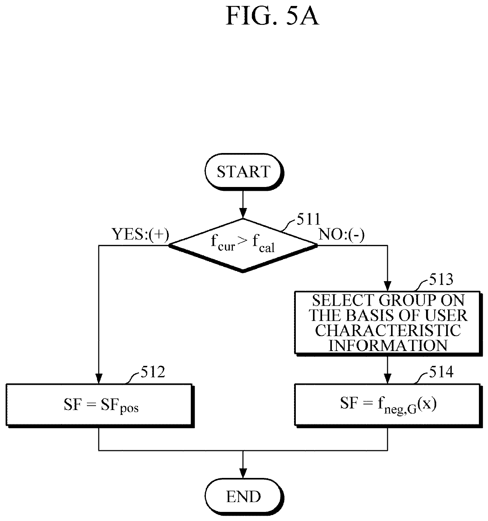

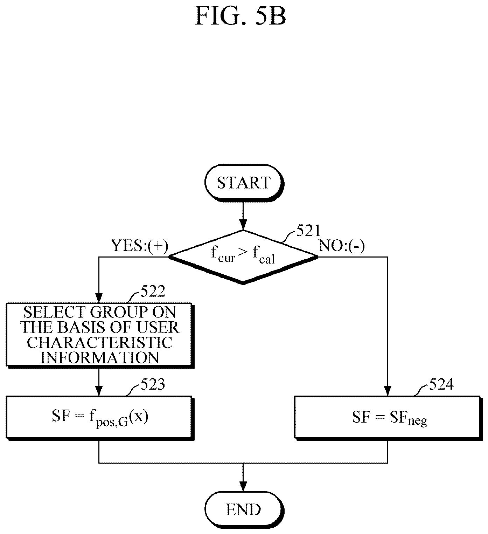

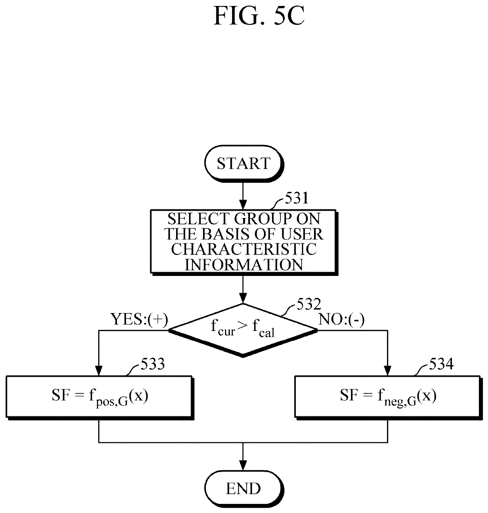

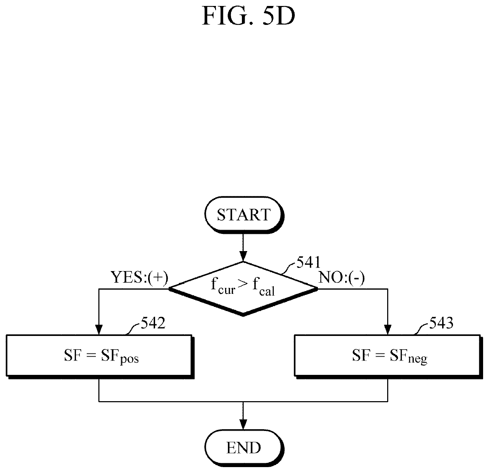

FIG. 4 is a flowchart illustrating a method of estimating blood pressure according to embodiments. FIGS. 5A, 5B, 5C and 5D are flowcharts illustrating acquisition of a scale factor according to embodiments. The embodiments of FIG. 4 to 5D may be embodiments of a method of estimating blood pressure that is performed by the apparatus 100a or 100b of FIG. 1A or 1B.

First, when the apparatus for estimating blood pressure receives a request for estimation of blood pressure, the apparatus may measure a bio-signal (410). The apparatus may provide an interface for interacting with a user and may receive the request for estimation of blood pressure from the user through the interface. Alternatively, the apparatus may receive a request for estimation of blood pressure from an external device. In this case, the request for estimation of blood pressure received from the external device may include a request for providing a blood pressure estimation result. When the external device is equipped with a blood pressure estimation algorithm, the request for estimation of blood pressure may include a request for providing an acquired feature. The external device may include a smartphone carried by the user, a tablet PC, a notebook PC, a wearable device, and the like. In this case, the bio-signal may include a PPG signal, but is not limited thereto.

Then, a feature value may be acquired from the bio-signal by analyzing the bio-signal (420). In this case, the feature value may be a value obtained by one or a combination of two or more of times and amplitudes at a maximum point and a minimum point of the bio-signal, time and amplitude at a position of a constituent pulse waveform, an area of the bio-signal, and a heart rate. In this case, a method of combining two or more of features is not particularly limited to a specific one.

Then, a blood pressure change sign may be detected and a scale factor may be acquired, using the acquired feature value (430). The blood pressure change sign may be detected based on a direction of change of the acquired feature value in operation 420 relative to a feature value at a reference point in time. Here, when there is no change in blood pressure, the blood pressure change sign is 0, but in this case, blood pressure at the reference point in time does not change, and hence it is described that the blood pressure change sign is limited to including only positive (+) and negative (-) signs.

Various embodiments of operation 420 of detecting the blood pressure change sign and acquiring a scale factor will be described with reference to FIGS. 5A to 5D. However, the embodiments are not limited to the examples described below.

Referring to FIG. 5A, the blood pressure change sign may be determined by comparing the feature value $f_{cur}$ acquired in operation 420 and a reference feature value $f_{cal}$ at the time of calibration (511).

Then, when it is determined in operation 511 that the blood pressure change sign is a positive (+) sign, indicating that the feature value $f_{cur}$ is greater than the reference feature value $f_{cal}$, a fixed constant value $SF_{pos}$ preset for the positive (+) sign may be acquired as a scale factor SF for scaling the feature value $f_{cur}$ (512). When it is determined in operation 511 that the blood pressure change sign is a negative (-) sign, indicating that the feature value $f_{cur}$ is smaller than the reference feature value $f_{cal}$, a group to which the user belongs may be selected on the basis of the user characteristic information (513). In this case, in a case in which a group is preset for a user, operation 513 may be omitted. Then, a scale factor SF may be acquired by applying a negative sign estimation equation $f_{neg,G}(x)$ that is preset for the group to which the user belongs (514).

Referring to FIG. 5B, the blood pressure change sign may be determined by comparing the feature value $f_{cur}$ acquired in operation 420 and the reference feature value $f_{cal}$ at the time of calibration (521).

Then, when it is determined in operation 521 that the blood pressure change sign is a positive (+) sign, indicating that the feature value $f_{cur}$ is greater than the reference feature value $f_{cal}$, a group to which the user belongs may be selected on the basis of the user characteristic information (522). In this case, when a group is preset for a user, operation 522 may be omitted. Then, a scale factor SF may be acquired by applying a positive (+) sign estimation equation $f_{pos,G}(x)$ that is preset for the group to which the user belongs (523). When it is determined in operation 521 that the blood pressure change sign is a negative (-) sign, indicating that the feature value $f_{cur}$ is smaller than the reference feature value $f_{cal}$, a fixed constant value $SF_{neg}$ preset for the negative (-) sign may be acquired as a scale factor SF (524).

Referring to FIG. 5C, a group to which the user belongs may be first selected on the basis of the user characteristic information (531). In this case, when a group is preset for a user, operation 531 may be omitted. Then, a blood pressure change sign may be determined by comparing the feature value $f_{cur}$ acquired in operation 420 and the reference feature value $f_{cal}$ at the time of calibration (532).

Then, when it is determined in operation 532 that the blood pressure change sign is a positive (+) sign, indicating that the feature value $f_{cur}$ is greater than the reference feature value $f_{cal}$, a scale factor SF may be acquired by applying a positive (+) sign estimation equation $f_{pos,G}(x)$ that is preset for the group to which the user belongs (533). When it is determined in operation 532 that the blood pressure change sign is a negative (-) sign, indicating that the feature value $f_{cur}$ is smaller than the reference feature value $f_{cal}$, a scale factor SF may be acquired by applying a negative (-) sign estimation equation $f_{neg,G}(x)$ that is preset for the group to which the user belongs (534).

Referring to FIG. 5D, the blood pressure change sign may be determined by comparing the feature value $f_{cur}$ acquired in operation 420 and the reference feature value $f_{cal}$ at the time of calibration (541).

Then, when it is determined operation 541 that the blood pressure change sign is a positive (+) sign, indicating that the feature value $f_{cur}$ is greater than the reference feature value $f_{cal}$, a fixed constant value $SF_{pos}$ preset for the positive (+) sign may be acquired as a scale factor SF (542). When it is determined that the blood pressure change sign is a negative (-) sign, indicating that the feature value $f_{cur}$ is smaller than the reference feature value $f_{cal}$, a fixed constant value $SF_{neg}$ preset for the negative (-) sign may be acquired as a scale factor SF (543).

Referring back to FIG. 4, blood pressure may be estimated on the basis of the feature value acquired in operation 420 and the scale factor acquired in operation 430 (440). For example, the feature value may be scaled using the scale factor as shown in the above Equation 1, and then blood pressure may be estimated by combining the scaled feature value with an offset value.

Then, a result of the estimation of the blood pressure may be output (450). For example, the blood pressure estimation result may be output through a display, using various visual methods. Alternatively, the blood pressure estimation result may be provided to the user through a speaker and/or a haptic module using a nonvisual method, such as voice, tactile sensation, vibration, or the like. In addition, the user's health status may be determined on the basis of estimated bio-information and warning or actions to be taken may be informed to the user according to a determination result.

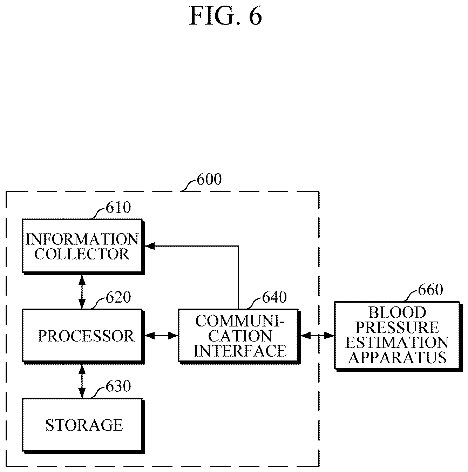

FIG. 6 is a block diagram illustrating an apparatus for supporting blood pressure estimation according to embodiments. The apparatus 600 for supporting blood pressure estimation may be mounted in a terminal, such as a smartphone, a tablet PC, a desktop PC, a notebook PC, or the like, a server of a manufacturer or a vendor of the apparatus 600, or a medical device manufactured for use in measuring and analyzing bio-information in a medical institution. The apparatus 600 for supporting blood pressure estimation may be mounted in such devices and may perform various functions for supporting a blood pressure estimation apparatus 660, for example, generating and updating a scale factor estimation model and distributing the scale factor estimation model to the blood pressure estimation apparatus 660.

Referring to FIG. 6, the apparatus 600 for supporting blood pressure estimation may include an information collector 610, a processor 620, a storage 630, and a communication interface 640.

The information collector 610 may collect information related to blood pressure of a plurality of users. For example, the information collector 610 may be connected through the communication interface 640 to the blood pressure estimation apparatus 660 used by the plurality of users and collect blood pressure-related information from the blood pressure estimation apparatus 660. Alternatively, the information collector 610 may directly collect information from a plurality of users who participate in a test by using various blood pressure-related devices including a cuff-type blood pressure measurement device. In this case, the blood pressure-related information may include bio-signals measured from the plurality of users, the above-described personal characteristic factor, an actual blood pressure measurement value obtained using a cuff blood pressure device or the like, etc., but is not limited thereto. The information collector 610 may be implemented by a processor.

The processor 620 may generate a scale factor estimation model associated with each blood pressure change sign for scaling a bio-signal feature value to be used in estimating blood pressure on the basis of the blood pressure-related information collected from the plurality of users. In this case, the scale factor estimation model may include a constant value that is defined differently for each blood pressure change sign as described above, an estimation equation that reflects characteristics of each individual, and an estimation equation that reflects characteristics of each group.

In one example, the processor 620 may classify the plurality of users according to the blood pressure change sign, acquire an optimal scale factor for each user on the basis of an actual blood pressure value and a bio-signal feature value of each user corresponding to the blood pressure change sign, and determine, on the basis of the acquired optimal scale factors, an optimal fixed constant value for each blood pressure change sign that is applicable to all users.

In another example, the processor 620 may classify the plurality of users according to the blood pressure change sign and acquire an optimal scale factor for each user on the basis of an actual blood pressure value and a bio-signal feature value of each user corresponding to each blood pressure change sign. For example, a scale factor may be acquired by inputting an actual blood pressure value, a baseline blood pressure value, and a bio-signal feature value into the above Equation 1. As such, by using the scale factor acquired for each user according to the blood pressure change sign and the personal characteristic factors of corresponding users, an estimation equation for each blood pressure change sign that reflects the characteristics of each individual may be obtained in the same manner as that described with reference to FIG. 3A.

In still another example, the processor 620 may classify the plurality of users according to the blood pressure change sign, and further sub-classify the classified users into groups according to sex, age group, whether the users take medication, occupational group, disease, or the like, and generate an estimation equation for each group, which reflects characteristics of the corresponding group. As described with reference to FIG. 3B, a scale factor estimation equation for each group may be acquired using the personal characteristic factors of users of each group and scale factors acquired for corresponding users.

The processor 620 may distribute the generated scale factor estimation model to the blood pressure estimation apparatus 660 through the communication interface 640. In a case in which there is a request from the blood pressure estimation apparatus 660 or in which it is determined that an update of a blood pressure estimation apparatus 600 is to be performed, the processor 620 may distribute the scale factor estimation model to the blood pressure estimation apparatus 660 when the generation of the scale factor estimation model is completed or when a preset period is reached.

The communication interface 640 may communicate with the blood pressure estimation apparatus 660 and receive blood pressure-related information and/or a request for the scale factor estimation model from the blood pressure estimation apparatus 660. In addition, the communication interface 640 may transmit the scale factor estimation model to the blood pressure estimation apparatus 660 under the control of the processor 620.

The storage 630 may store the blood pressure-related information collected from the blood pressure estimation apparatus 660 or collected through a test. In addition, when the scale factor estimation model is generated by the processor 620, the storage 630 may store the generated scale factor estimation model.

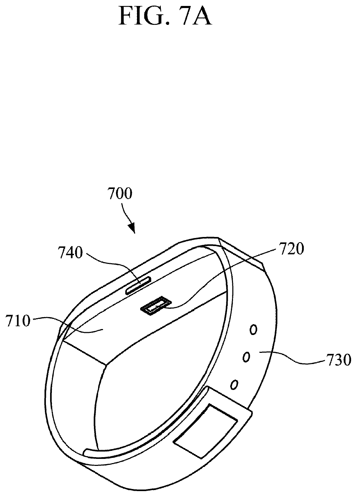

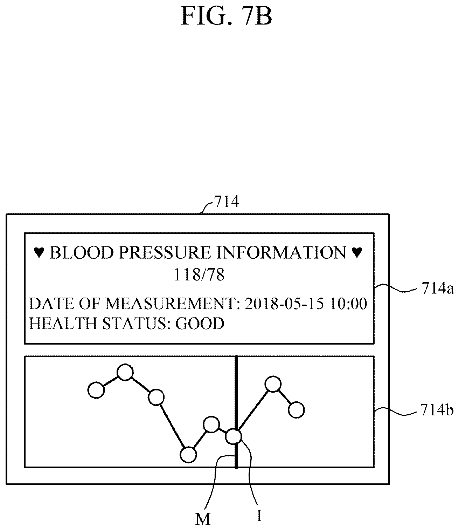

FIGS. 7A and 7B are diagrams illustrating a wearable device. The above-described embodiments of the apparatuses 100a and 100b for estimating blood pressure may be mounted in a smart watch worn on a wrist or a smart band-type wearable device. However, such devices are examples for convenience of description, and the embodiments may be applied to an information processing terminal, such as a smartphone, a tablet PC, a notebook PC, a desktop PC, or the like.

Referring to FIGS. 7A and 7B, the wearable device 700 may include a device main body 710 and a strap 730.

The main body 710 may be configured in various forms and may have modules mounted inside or on a surface thereof to perform a blood pressure estimation function and other various functions. A battery may be embedded inside of the main body 710 or the strap to supply power to various modules of the device 700.

The strap 730 may be connected to the main body 710. The strap 730 may be formed to be flexible to be bent in a shape to wrap around a wrist of a user. The strap 730 may be configured in a form that is detached from the user's wrist or be configured in the form of an undivided band. The strap 730 may be filled with air or have an air bag to have elasticity according to a change in pressure applied to the wrist and may transmit the pressure change of the wrist to the main body 710.

A sensor 720 configured to measure a bio-signal may be mounted in the main body 710. The sensor 720 may be mounted on a rear surface of the main body 710 that is brought into contact with an upper part of the user's wrist, and may include a light source configured to emit light to the wrist skin and a detector configured to detect light scattered or reflected from an object of interest. The sensor 720 may further include a contact pressure sensor configured to measure a contact pressure exerted by the object of interest.

A processor may be mounted inside of the main body 710 and the processor may be electrically connected to each configuration mounted in the wearable device 700 to control each configuration. In addition, the processor may estimate blood pressure using the bio-signal measured by the sensor 720. The processor may acquire a feature value from the bio-signal, as described above, and detect a blood pressure change sign using the acquired feature value. In addition, the processor may acquire a scale factor by applying a scale factor estimation model preset for each blood pressure change sign and estimate blood pressure after scaling the feature value using the acquired scale factor.

In the case in which the contact pressure sensor is mounted, the processor may monitor a contact state of the object of interest on the basis of a contact pressure between the wrist and the sensor 720 and may inform the user of a contact position and/or a contact state through a display.

In addition, a storage configured to store processing results of the processor and a variety of information may be mounted inside the main body 710. In this case, the variety of information may include reference information for blood pressure estimation and various pieces of information related to functions of the wearable device 700.

Moreover, an operator 740 configured to receive a control instruction of the user and transmit the control instruction to the processor may be mounted in the main body 710. The operator 740 may include a power button for inputting an instruction for turning on/off the wearable device 700.

A display 714 may be mounted on a front surface of the main body 710 and may include a touch panel capable of receiving touch inputs. The display 714 may receive a user's touch input, transmit the touch input to the processor, and display processing results of the processor.

For example, the display 714 may display estimated blood pressure information. In this case, additional information, such as date of estimation of blood pressure or health status, may be displayed together with the blood pressure information. At this time, if the user requests detailed information by manipulating the operator 740 or through touch input to the display 714, the detailed information may be output in various ways.

Referring to FIG. 7B, the display 714 may output the detailed information in a first region 714a and output a blood pressure history graph in a second region 714b. In this case, the blood pressure history graph may include an object (e.g., a figure, such as a circle, a rectangle, or the like) indicating the time of blood pressure estimation. In addition, an identification mark M that indicates an object I currently selected by the user may be displayed on the blood pressure history graph. Although the identification mark I is illustrated as a vertical line, the embodiment is not limited thereto and the identification mark I may be displayed in various forms, such as a circle, a polygon, such as a rectangle, an arrow indicating a pertinent position, and the like. The user may request the display of the blood pressure history graph. When the blood pressure graph is displayed in the second region 714b, the user may touch an object I at a point in time or move the graph to the left or right to match the object I at the point in time to the identification mark M so that the detailed information can be output in the first region 714a. In this case, information, such as an estimated blood pressure value at the estimation time selected by the user, the measurement date, and the health status at the pertinent point in time may be output to the first region 714a, but the information is not limited thereto.

In addition, a communication interface configured to communicate with an external device, such as a portable terminal of the user, may be mounted in the main body 710. The communication interface may transmit a result of estimation of bio-information to the external device, for example, a smartphone of the user, and allow the result to be displayed to the user. However, the embodiment is not limited thereto and a variety of information may be transmitted and received.

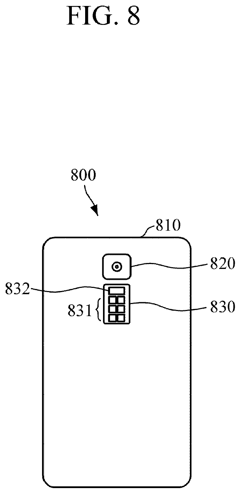

FIG. 8 is a diagram illustrating a smart device to which the embodiments of the apparatus for estimating blood pressure are applied. In this case, the smart device may include a smartphone, a tablet PC, and the like.

Referring to FIG. 8, a sensor 830 may be mounted on one surface of a main body 810 of a smart device 800. The sensor 830 may include a pulse wave sensor including one or more light sources 831 and a detector 832. As shown in FIG. 8, the sensor 830 may be mounted on a rear surface of the main body 810, but is not limited thereto. The sensor 830 may be combined with a fingerprint sensor or a touch panel on a front surface of the main body 810.

In addition, a display may be mounted on the front surface of the main body 810. The display may visually output a result of estimation of bio-information or the like. The display may include a touch panel and receive and transmit a variety of information input through the touch panel to a processor.

An image sensor 820 may be mounted in the main body 810. When the user approaches a finger to the sensor 830 to measure a pulse wave signal, the image sensor 820 may capture a finger image and transmit it to the processor. In this case, the processor may identify a relative position of the finger relative to an actual position of the sensor 830 from the finger image and provide the relative position information of the finger to the user through the display, thereby guiding the user to more accurately measure the pulse wave signal.

When the bio-signal is measured by the sensor 830 as described above, the processor may estimate blood pressure in consideration of a blood pressure change sign relative to a reference point in time. Various modules for performing other functions may be mounted in the smart device 800 and detailed descriptions thereof will be omitted.

The current embodiments can be implemented as computer readable codes in a computer readable record medium. Codes and code segments constituting the computer program can be easily inferred by a skilled computer programmer in the art. The computer readable record medium includes all types of record media in which computer readable data are stored. Examples of the computer readable record medium include a ROM, a RAM, a CD-ROM, a magnetic tape, a floppy disk, and an optical data storage. Further, the record medium may be implemented in the form of a carrier wave such as Internet transmission. In addition, the computer readable record medium may be distributed to computer systems over a network, in which computer readable codes may be stored and executed in a distributed manner.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents.

**Claims**

1. An apparatus for estimating a blood pressure, the apparatus comprising:

   a sensor (110, 720, 820) configured to measure (410) a bio-signal; and
   a processor (120, 620) configured to:

      acquire (420) a feature value from the bio-signal;
      detect (430) a blood pressure change sign, based on the feature value;
      acquire a scale factor, based on the blood pressure change sign; and
      estimate (440) the blood pressure, based on the feature value and the scale factor,

   **characterized in that**
   the processor (120, 620) is further configured to detect the blood pressure change sign, based on whether the acquired feature value is greater than a reference feature value being acquired from the bio-signal measured at a time of calibration, when a preset condition for performing calibration is satisfied.

2. The apparatus of claim 1, wherein the processor (120, 620) is further configured to:
   detect that the blood pressure change sign is positive, based on the acquired feature value being greater than the reference feature value; and detect that the blood pressure change sign is negative, based on the acquired feature value being less than the reference feature value.

3. The apparatus of claim 1 or 2, wherein the processor (120, 620) is further configured to acquire the scale factor, using a scale factor estimation model that is defined differently according to the blood pressure change sign,

   wherein the scale factor estimation model comprises any one or any combination of a constant value, a first estimation equation reflecting characteristics of users, and a second estimation equation reflecting characteristics of each of groups of the users,
   wherein the processor (120, 620) is further configured to:

      acquire the scale factor as a first constant value that is defined for a positive sign, based on the blood pressure change sign being detected to be positive; and
      acquire the scale factor as a second constant value that is defined for a negative sign, based on the blood pressure change sign being detected to be negative.

4. The apparatus of claim 3, wherein the processor (120, 620) is further configured to:

   acquire the scale factor as the constant value that is defined for a positive sign, based on the blood pressure change sign being detected to be positive; and
   acquire the scale factor, using the second estimation equation that is defined for a negative sign, based on the blood pressure change sign being detected to be negative.

5. The apparatus of claim 4, wherein the processor (120, 620) is further configured to, based on the blood pressure change sign being determined to be negative:

   receive characteristic information of one of the users; and
   acquire the scale factor, based on the characteristic information being applied to the second estimation equation for one of the groups to which the one of the users belongs.

6. The apparatus of claim 4, wherein each of the groups is classified based on any one or any combination of sex, age, whether medication is taken, occupation, and disease, or wherein each of the first estimation equation and the second estimation equation has an input of a personal characteristic factor reflecting at least one of the characteristics of one of the users, and wherein the personal characteristic factor comprises any one or any combination of an age, a sex, a height, a weight, a body mass index, a pulse pressure, a baseline systolic blood pressure, a baseline diastolic blood pressure, a difference between the baseline systolic blood pressure and the baseline diastolic blood pressure, and a heart rate.

7. The apparatus of one of claims 1 to 6, wherein the processor (120, 620) is further configured to acquire the feature value by combining one or more of a shape of a waveform of the bio-signal, a time and an amplitude at a maximum point of the bio-signal, a time and an amplitude at a minimum point of the bio-signal, a time and an amplitude at a position of a pulse waveform component constituting the bio-signal, and an area of the bio-signal,
or
wherein the processor (120, 620) is further configured to scale the feature value with the scale factor; and estimate the blood pressure, based on the scaled feature value, wherein the processor is further configured to estimate the blood pressure, further based on a baseline blood pressure value at a time of calibration.

8. The apparatus of one of claims 1 to 7, wherein the sensor (110, 720, 820) comprises: a light source configured to emit light to an object of interest; and a detector configured to detect light that is scattered from the object of interest, or further comprising an output interface configured to output a processing result of the processor.

9. The apparatus of one of claims 1 to 8, further comprising a communication interface configured to receive a scale factor estimation model to be used to acquire the scale factor, from an external device,
wherein the processor is further configured to:

   determine whether to update the scale factor estimation model, based on any one or any combination of a preset period, a change in characteristics of a user, and
   the estimated blood pressure; and control the communication interface to receive a new scale factor estimation model from the external device, based on the scale factor estimation model being determined to be updated.

10. A method of operating an apparatus for estimating a blood pressure, the method comprising:

   measuring (410), by a sensor (110, 720, 820), a bio-signal;
   acquiring (420), by a processor (120, 620) a feature value from the bio-signal;
   detecting (430), by the processor (120, 620) a blood pressure change sign, based on the feature value;
   acquiring, by the processor (120, 620) a scale factor, based on the blood pressure change sign; and
   estimating (440), by the processor (120, 620) the blood pressure, based on the feature value and the scale factor, **characterized in that**
   the step of detecting (430) the blood pressure change sign comprises detecting the blood pressure change sign, based on whether the acquired feature value is greater than a reference feature value being acquired from the bio-signal measured at a time of calibration, when a preset condition for performing calibration is satisfied.

11. The method of claim 10 further comprising generating, by the processor (120, 620), based on the blood pressure-related information, a scale factor estimation model for each of a positive blood pressure change sign and a negative blood pressure change sign, the scale factor estimation model being for scaling a bio-signal feature value to be used in estimating a blood pressure,
wherein the scale factor estimation model comprises any one or any combination of a constant value, a first estimation equation reflecting characteristics of the users, and a second estimation equation reflecting characteristics of each of groups of the users, and further comprising:

   classifying, by the processor (120, 620), the users, based on a blood pressure change sign of a bio-signal feature value for each of the users; acquiring, by the processor, an optimal scale factor of each of the users, based on the bio-signal feature value and an actual blood pressure value of each of the users, the bio-signal feature value and the actual blood pressure value corresponding to the blood pressure change sign of each of the users; and generating, by the processor, the first estimation equation reflecting the characteristics of the users, based on a
   personal characteristic factor and the optimal scale factor of each of the users, or further comprising:

classifying, by the processor (120, 620), the users, based on a blood pressure change sign of a bio-signal feature value for a respective one of the users; sub-classifying, by the processor, the classified users into the groups, based on any one or any combination of sex, age, whether medication is taken, occupation, and disease; and generating, by the processor, the second estimation equation reflecting the characteristics of each of the groups.

12. The method of claim 10 or 11, further comprising transmitting, by a communication interface, the scale factor estimation model to an apparatus for estimating blood pressure, based on a request being received from the apparatus for estimating blood pressure or based on the scale factor estimation model being generated.

13. The method of one of claims 10 to 12, further comprising storing, by a storage, either one or both of the blood pressure-related information and the scale factor estimation model.

14. A computer-readable medium having instructions that, when performed by a processor (120, 620) of an apparatus for estimating a blood pressure having a sensor (110, 720, 820) configured to measure (410) a bio-signal of a user, cause the processor (120, 620) to:

acquire (420) a current feature value from the bio-signal;
determine whether the current feature value is greater than a reference feature value;
based on the current feature value being determined to be greater than the reference feature value, detect (430) a blood pressure change sign based on the feature value;
acquire a scale factor based on the blood pressure change sign;
scale the current feature value with the scale factor; and
estimate (440) the blood pressure, based on the scaled current feature value,
wherein the processor (120, 620) is further configured to detect the blood pressure change sign, based on whether the acquired feature value is greater than a reference feature value being acquired from the bio-signal measured at a time of calibration, when a preset condition for performing calibration is satisfied.

15. The computer-readable medium of claim 14, having instructions to further cause the processor (120, 620) to:

based on the current feature value being determined to be greater than the reference feature value: select a group to which the user belongs, based on characteristic information of the user; and acquire the scale factor, using an estimation equation that is preset for the selected group and for when the current feature value being determined to be greater than the reference feature value; and based on the current feature value being determined to be less than the reference feature value, setting the scale factor to a value that is preset for when the current feature value is determined to be less than the reference feature value, or
having instructions to further cause the processor (120, 620) to: select a group to which the user belongs, based on characteristic information of the user; based on the current feature value being determined to be greater than the reference feature value, acquire the scale factor, using a first estimation equation that is preset for the selected group and for when the current feature value being determined to be greater than the reference feature value; and based on the current feature value being determined to be less than the reference feature value, acquire the scale factor, using a second estimation equation that is preset for the selected group and for when the current feature value being determined to be less than the reference feature value.

**Patentansprüche**

1. Vorrichtung zum Schätzen eines Blutdrucks, wobei die Vorrichtung umfasst:

einen Sensor (110, 720, 820) konfiguriert zum Messen (410) eines Biosignals; und
einen Prozessor (120, 620) konfiguriert zum:

Erfassen (420) eines Merkmalswertes aus dem Biosignal;
Detektieren (430) eines Vorzeichens für eine Blutdruckänderung, basierend auf dem Merkmalswert;
Erfassen eines Skalierungsfaktors, basierend auf dem Vorzeichen der Blutdruckänderung; und
Schätzen (440) des Blutdrucks, basierend auf dem Merkmalswert und dem Skalierungsfaktor,

**dadurch gekennzeichnet, dass**

der Prozessor (120, 620) ferner konfiguriert ist, um das Vorzeichen der Blutdruckänderung zu detektieren, basierend darauf, ob der erfasste Merkmalswert größer ist als ein Referenzmerkmalswert, der von dem Biosignal erfasst wird, das zu einem Zeitpunkt der Kalibrierung gemessen wird, wenn eine voreingestellte Bedingung für die Durchführung der Kalibrierung erfüllt ist.

2. Vorrichtung nach Anspruch 1, wobei der Prozessor (120, 620) ferner konfiguriert ist, zum:

Detektieren, dass das Vorzeichen der Blutdruckänderung positiv ist, basierend darauf, dass der erfasste Merkmalswert größer als der Referenzmerkmalswert ist; und
Detektieren, dass das Vorzeichen der Blutdruckänderung negativ ist, basierend darauf, dass der erfasste Merkmalswert kleiner als der Referenzmerkmalswert ist.

3. Vorrichtung nach Anspruch 1 oder 2, wobei der Prozessor (120, 620) ferner konfiguriert ist zum Erfassen des Skalenfaktors unter Verwendung eines Skalenfaktor-Schätzmodells, das entsprechend dem Vorzeichen der Blutdruckänderung unterschiedlich definiert ist,

wobei das Skalenfaktor-Schätzmodell eine beliebige oder eine beliebige Kombination aus einem konstanten Wert, einer ersten Schätzungsgleichung, die Merkmale von Benutzern widerspiegelt, und einer zweiten Schätzungsgleichung, die Merkmale jeder der Gruppen der Benutzer widerspiegelt, umfasst,
wobei der Prozessor (120, 620) weiterhin konfiguriert ist, um:
den Skalierungsfaktor als einen ersten konstanten Wert zu erfassen, der für ein positives Vorzeichen definiert ist, basierend darauf, dass das Vorzeichen der Blutdruckänderung als positiv erfasst wird; und den Skalierungsfaktor als einen zweiten konstanten Wert zu erfassen, der für ein negatives Vorzeichen definiert ist, basierend darauf, dass das Vorzeichen der Blutdruckänderung als negativ erfasst wird.

4. Vorrichtung nach Anspruch 3, wobei der Prozessor (120, 620) ferner konfiguriert ist, zum:

Erfassen des Skalierungsfaktors als den konstanten Wert, der für ein positives Vorzeichen definiert ist, basierend darauf, dass das Vorzeichen der Blutdruckänderung als positiv erfasst wird; und
Erfassen des Skalierungsfaktors unter Verwendung der zweiten Schätzgleichung, die für ein negatives Vorzeichen definiert ist, basierend darauf, dass das Vorzeichen der Blutdruckänderung als negativ erfasst wird.

5. Vorrichtung nach Anspruch 4, wobei der Prozessor (120, 620) ferner konfiguriert ist, um basierend auf dem als negativ ermittelten Vorzeichen der Blutdruckänderung charakteristische Informationen eines der Benutzer zu empfangen; und
Erfassen des Skalierungsfaktors basierend auf der charakteristischen Information, die auf die zweite Schätzungsgleichung für eine der Gruppen angewendet wird, zu der der eine der Benutzer gehört.

6. Vorrichtung nach Anspruch 4, wobei jede der Gruppen basierend auf einer oder einer Kombination von Geschlecht, Alter, ob Medikamente eingenommen werden, Beruf und Krankheit klassifiziert wird, oder
wobei sowohl die erste Schätzgleichung als auch die zweite Schätzgleichung eine Eingabe eines persönlichen charakteristischen Faktors aufweist, der mindestens eines der Merkmale eines der Benutzer widerspiegelt, und wobei der persönliche charakteristische Faktor eine beliebige oder eine beliebige Kombination aus einem Alter, einem Geschlecht, einer Größe, einem Gewicht, einem Body-Maß-Index, einem Pulsdruck, einem systolischen Grundlinienblutdruck, einem diastolischen Grundlinienblutdruck, einer Differenz zwischen dem systolischen Grundlinienblutdruck und dem diastolischen Grundlinienblutdruck und einer Herzfrequenz umfasst.

7. Vorrichtung nach einem der Ansprüche 1 bis 6, wobei der Prozessor (120, 620) ferner konfiguriert ist zum Erfassen des Merkmalswerts durch Kombinieren einer oder mehrerer aus einer Form einer Wellenform des Biosignals, einer Zeit und einer Amplitude an einem Maximalpunkt des Biosignals, einer Zeit und einer Amplitude an einem Minimalpunkt des Biosignals, einer Zeit und einer Amplitude an einer Position einer Pulswellenformkomponente, die das Biosignal bildet, und einer Fläche des Biosignals, oder
wobei der Prozessor (120, 620) ferner konfiguriert ist zum Skalieren des Merkmalswerts mit dem Skalierungsfaktor; und zum Schätzen des Blutdrucks basierend auf dem skalierten Merkmalswert, wobei der Prozessor ferner konfiguriert ist zum Schätzen des Blutdrucks, ferner basierend auf einem Basisblutdruckwert zu einem Zeitpunkt der Kalibrierung.

8. Vorrichtung nach einem der Ansprüche 1 bis 7, wobei der Sensor (110, 720, 820) umfasst:

eine Lichtquelle konfiguriert zum Aussenden von Licht auf ein Objekt von Interesse; und einen Detektor konfiguriert zum Erfassen von Licht, das von dem Objekt von Interesse gestreut wird, oder

ferner umfassend eine Ausgabeschnittstelle, die konfiguriert ist, um ein Verarbeitungsergebnis des Prozessors auszugeben.

9. Vorrichtung nach einem der Ansprüche 1 bis 8 ferner umfassend eine Kommunikationsschnittstelle, die konfiguriert ist, um ein Skalenfaktor-Schätzmodell, das zur Erfassung des Skalenfaktors verwendet werden soll, von einer externen Vorrichtung zu empfangen,

wobei der Prozessor weiterhin konfiguriert ist, zum:

Bestimmen, ob das Skalenfaktor-Schätzmodell zu aktualisieren ist, basierend auf einer oder einer Kombination aus einer voreingestellten Periode, einer Änderung der Eigenschaften eines Benutzers und dem geschätzten Blutdruck; und Steuern der Kommunikationsschnittstelle, um ein neues Skalenfaktor-Schätzmodell von der externen Vorrichtung zu empfangen, basierend auf dem Skalenfaktor-Schätzmodell, das als zu aktualisieren bestimmt ist.

10. Verfahren zum Betreiben einer Vorrichtung zum Schätzen eines Blutdrucks, wobei das Verfahren umfasst:

Messen (410) eines Biosignals durch einen Sensor (110, 720, 820);
Erfassen (420), durch einen Prozessor (120, 620), eines Merkmalswertes aus dem Biosignal;
Detektieren (430), durch den Prozessor (120, 620), eines Vorzeichens für eine Blutdruckänderung, basierend auf dem Merkmalswert;
Erfassen eines Skalierungsfaktors durch den Prozessor (120, 620) basierend auf dem Vorzeichen der Blutdruckänderung; und
Schätzen (440), durch den Prozessor (120, 620), des Blutdrucks, basierend auf dem Merkmalswert und dem Skalenfaktor,
**dadurch gekennzeichnet, dass**
der Schritt des Detektierens (430) des Vorzeichens der Blutdruckänderung das Detektieren des Vorzeichens der Blutdruckänderung umfasst, basierend darauf, ob der erfasste Merkmalswert größer ist als ein Referenzmerkmalswert, der von dem Biosignal erfasst wird, das zu einem Zeitpunkt der Kalibrierung gemessen wird, wenn eine voreingestellte Bedingung zum Durchführen der Kalibrierung erfüllt ist.

11. Verfahren nach Anspruch 10 ferner umfassend das Erzeugen eines Skalierungsfaktor-Schätzmodells durch den Prozessor (120, 620) basierend auf den blutdruckbezogenen Informationen für jedes aus einem positiven Vorzeichen einer Blutdruckänderung und einem negativen Vorzeichen einer Blutdruckänderung, wobei das Skalierungsfaktor-Schätzmodell zum Skalieren eines Biosignal-Merkmalswerts dient, der beim Schätzen eines Blutdrucks zu verwenden ist,

wobei das Skalierungsfaktor-Schätzmodell eine beliebige oder eine beliebige Kombination aus einem konstanten Wert, einer ersten Schätzungsgleichung, die Merkmale der Benutzer widerspiegelt, und einer zweiten Schätzungsgleichung, die Merkmale jeder der Gruppen der Benutzer widerspiegelt, umfasst, und
ferner umfassend:

Klassifizieren der Benutzer durch den Prozessor (120, 620) basierend auf einem Vorzeichen der Blutdruckänderung eines Biosignal-Merkmalswertes für jeden der Benutzer; Erfassen durch den Prozessor eines optimalen Skalierungsfaktors für jeden der Benutzer basierend auf dem Biosignal-Merkmalswert und einem tatsächlichen Blutdruckwert für jeden der Benutzer, wobei der Biosignal-Merkmalswert und der tatsächliche Blutdruckwert dem Vorzeichen der Blutdruckänderung für jeden der Benutzer entsprechen; und Erzeugen, durch den Prozessor, der ersten Schätzungsgleichung, die die Eigenschaften der Benutzer widerspiegelt, basierend auf einem persönlichen Eigenschaftsfaktor und dem optimalen Skalierungsfaktor jedes der Benutzer, oder
ferner umfassend:
Klassifizieren durch den Prozessor (120, 620) der Benutzer basierend auf einem Vorzeichen der Blutdruckänderung eines Biosignal-Merkmalswertes für einen jeweiligen der Benutzer; Unterklassifizieren durch den Prozessor der klassifizierten Benutzer in die Gruppen basierend auf eine beliebige oder eine beliebige Kombination aus Geschlecht, Alter, ob Medikamente eingenommen werden, Beruf und Krankheit; und Erzeugen durch den Prozessor der zweiten Schätzungsgleichung, die die Eigenschaften jeder der Gruppen widerspiegelt.

12. Verfahren nach Anspruch 10 oder 11 ferner umfassend das Übertragen des Skalenfaktor-Schätzmodells durch eine Kommunikationsschnittstelle an eine Vorrichtung zum Schätzen des Blutdrucks basierend auf einer Anforderung, die von der Vorrichtung zum Schätzen des Blutdrucks empfangen wird, oder basierend auf dem erzeugten Skalenfaktor-Schätzmodell.

13. Verfahren nach einem der Ansprüche 10 bis 12 ferner umfassend Speichern entweder einer oder beider der blutdruckbezogenen Informationen und des Skalenfaktor-Schätzmodells durch einen Storage.

14. Computerlesbares Medium mit Instruktionen, die, wenn sie von einem Prozessor (120, 620) einer Vorrichtung zum Schätzen eines Blutdrucks mit einem Sensor (110, 720, 820) ausgeführt werden, der konfiguriert ist, um ein Biosignal eines Benutzers zu messen (410), den Prozessor (120, 620) veranlassen, folgende Schritte auszuführen:

Erfassen (420) eines aktuellen Merkmalswertes aus dem Biosignal;
Bestimmen, ob der aktuelle Merkmalswert größer ist als ein Referenzmerkmalswert;
basierend auf dem aktuellen Merkmalswert, der als größer als der Referenzmerkmalswert bestimmt wird, Detektieren (430) eines Vorzeichens einer Blutdruckänderung basierend auf dem Merkmalswert;
Erfassen eines Skalierungsfaktors basierend auf dem Vorzeichen der Blutdruckänderung;
Skalieren des aktuellen Merkmalswertes mit dem Skalierungsfaktor; und
Schätzen (440) des Blutdrucks basierend auf dem skalierten aktuellen Merkmalswert,

wobei der Prozessor (120, 620) ferner konfiguriert ist, um das Vorzeichen der Blutdruckänderung zu erfassen, basierend darauf, ob der erfasste Merkmalswert größer ist als ein Referenzmerkmalswert, der von dem Biosignal erfasst wird, das zu einer Zeit der Kalibrierung gemessen wird, wenn eine voreingestellte Bedingung zur Durchführung der Kalibrierung erfüllt ist.

15. Computerlesbares Medium nach Anspruch 14, mit Instruktionen, die den Prozessor (120, 620) ferner veranlassen, folgende Schritte auszuführen:

basierend auf dem aktuellen Merkmalswert, der als größer als der Referenzmerkmalswert bestimmt wird: Auswählen einer Gruppe, zu der der Benutzer gehört, basierend auf charakteristischen Informationen des Benutzers; und Erfassen des Skalierungsfaktors unter Verwendung einer Schätzgleichung, die für die ausgewählte Gruppe und für den Fall voreingestellt ist, dass der aktuelle Merkmalswert als größer als der Referenzmerkmalswert bestimmt wird; und basierend auf dem aktuellen Merkmalswert, der als kleiner als der Referenzmerkmalswert bestimmt wird, Einstellen des Skalierungsfaktors auf einen Wert, der für den Fall voreingestellt ist, dass der aktuelle Merkmalswert als kleiner als der Referenzmerkmalswert bestimmt wird, oder
Instruktionen zu haben, um den Prozessor (120, 620) weiterhin zu veranlassen: Auswählen einer Gruppe, zu der der Benutzer gehört, basierend auf charakteristischen Informationen des Benutzers; basierend auf dem aktuellen Merkmalswert, der als größer als der Referenzmerkmalswert bestimmt wird, Erfassen des Skalierungsfaktors unter Verwendung einer ersten Schätzgleichung, die für die ausgewählte Gruppe und für den Fall voreingestellt ist, dass der aktuelle Merkmalswert als größer als der Referenzmerkmalswert bestimmt wird; und basierend auf dem aktuellen Merkmalswert, der als kleiner als der Referenzmerkmalswert bestimmt wird, Erfassen des Skalierungsfaktors unter Verwendung einer zweiten Schätzgleichung, die für die ausgewählte Gruppe und für den Fall voreingestellt ist, dass der aktuelle Merkmalswert als kleiner als der Referenzmerkmalswert bestimmt wird.

**Revendications**

1. Appareil d'estimation de la pression artérielle, comprenant :

un capteur (110, 720, 820) configuré pour mesurer (410) un biosignal ; et
un processeur (120, 620) configuré pour :

acquérir (420) une valeur de caractéristique du signal biologique ;
détecter (430) un signe de changement de la pression artérielle, sur la base de la valeur de la caractéristique ;
acquérir un facteur d'échelle, sur la base du signe de changement de la pression artérielle ; et
estimer (440) la pression artérielle, sur la base de la valeur de la caractéristique et du facteur d'échelle,
**caractérisé en ce que** le processeur (120,620) est en outre configuré pour détecter le signe de changement de la pression artérielle, sur la base du fait que la valeur de la caractéristique acquise est supérieure à une valeur de la caractéristique de référence acquise à partir du biosignal mesuré au moment de l'étalonnage, lorsqu'une condition prédéfinie pour la réalisation de l'étalonnage est remplie.

2. Appareil selon la revendication 1, dans lequel le processeur (120, 620) est en outre configuré pour :

détecter que le signe de changement de la pression artérielle est positif, sur la base de la valeur de caractéristique acquise supérieure à la valeur de caractéristique de référence ; et
détecter que le signe de changement de la pression artérielle est négatif, sur la base de la valeur de caractéristique acquise inférieure à la valeur de caractéristique de référence.

3. Appareil selon les revendications 1 ou 2, dans lequel le processeur (120, 620) est en outre configuré pour acquérir le facteur d'échelle, en utilisant un modèle d'estimation du facteur d'échelle qui est défini différemment en fonction du signe de changement de la pression artérielle,

dans lequel le modèle d'estimation du facteur d'échelle comprend une valeur constante ou une combinaison de valeurs constantes, une première équation d'estimation reflétant les caractéristiques des utilisateurs, et une seconde équation d'estimation reflétant les caractéristiques de chacun des groupes d'utilisateurs,
dans lequel le processeur (120, 620) est en outre configuré pour :

acquérir le facteur d'échelle en tant que première valeur constante définie pour un signe positif, sur la base du signe de changement de la pression artérielle détecté comme étant positif ; et
acquérir le facteur d'échelle en tant que seconde valeur constante définie pour un signe négatif, sur la base du signe de changement de la pression artérielle détecté comme étant négatif.

4. Appareil selon la revendication 3, dans lequel le processeur (120, 620) est en outre configuré pour :

acquérir le facteur d'échelle en tant que valeur constante définie pour un signe positif, sur la base du signe de changement de la pression artérielle détecté comme étant positif ; et
acquérir le facteur d'échelle, en utilisant la seconde équation d'estimation définie pour un signe négatif, sur la base du signe de changement de la pression artérielle détecté comme étant négatif.

5. Appareil selon la revendication 4, dans lequel le processeur (120, 620) est en outre configuré pour, en fonction du signe de changement de la pression artérielle déterminé comme étant négatif :

recevoir des informations sur les caractéristiques de l'un des utilisateurs ; et
acquérir le facteur d'échelle, sur la base des informations caractéristiques appliquées à la seconde équation d'estimation pour l'un des groupes auxquels appartient l'un des utilisateurs.

6. Appareil de la revendication 4, dans lequel chacun des groupes est classé sur la base d'une combinaison quelconque de sexe, d'âge, de prise de médicaments, de profession et de maladie, ou dans lequel la première équation d'estimation et la seconde équation d'estimation comportent un facteur de caractéristique personnelle reflétant au moins l'une des caractéristiques de l'un des utilisateurs, et dans lequel le facteur de caractéristique personnelle comprend l'un des éléments suivants ou une combinaison de ceux-ci : l'âge, le sexe, la taille, le poids, l'indice de masse corporelle, le pouls, la pression artérielle systolique de base, la pression artérielle diastolique de base, la différence entre la pression artérielle systolique de base et la pression artérielle diastolique de base, et la fréquence cardiaque.

7. Appareil selon l'une des revendications 1 à 6, dans lequel le processeur (120, 620) est en outre configuré pour acquérir la valeur de la caractéristique en combinant un ou plusieurs éléments parmi une forme d'onde du signal biologique, un temps et une amplitude à un point maximum du signal biologique, un temps et une amplitude à un point minimum du signal biologique, un temps et une amplitude à une position d'une composante de forme d'onde d'impulsion constituant le signal biologique, et une zone du signal biologique,
ou
dans lequel le processeur (120, 620) est en outre configuré pour mettre à l'échelle la valeur de la caractéristique avec le facteur d'échelle ; et estimer la pression artérielle sur la base de la valeur de la caractéristique mise à l'échelle, le processeur étant en outre configuré d'estimation de la pression artérielle sur la base d'une valeur de base de la pression artérielle au moment de l'étalonnage.

8. Appareil selon l'une des revendications 1 à 7, dans lequel le capteur (110, 720, 820) comprend :

une source lumineuse configurée pour émettre de la lumière vers un objet d'intérêt ; et
un détecteur configuré pour détecter la lumière diffusée par l'objet d'intérêt, ou
comprenant en outre une interface de sortie configurée pour émettre un résultat de traitement du processeur.

9. Appareil selon l'une des revendications 1 à 8, comprenant en outre une interface de communication configurée pour recevoir un modèle d'estimation de facteur d'échelle à utiliser pour acquérir le facteur d'échelle, à partir d'un dispositif externe,
dans lequel le processeur est en outre configuré pour :

déterminer s'il faut mettre à jour le modèle d'estimation du facteur d'échelle, sur la base de l'une ou l'autre combinaison d'une période prédéfinie, d'un changement dans les caractéristiques d'un utilisateur et de la pression artérielle estimée ; et
commander l'interface de communication pour recevoir un nouveau modèle d'estimation du facteur d'échelle du dispositif externe, sur la base du modèle d'estimation du facteur d'échelle déterminé comme devant être mis à jour.

10. Procédé de fonctionnement d'un appareil permettant d'estimer la pression artérielle, comprenant les opérations suivantes :

mesurer (410), par un capteur (110, 720, 820), un biosignal ;
acquérir (420), à l'aide du processeur (120, 620), une valeur caractéristique du signal biologique ;
détecter (430), à l'aide du processeur (120, 620), un signe de changement de la pression artérielle, sur la base de la valeur caractéristique ;
acquérir, à l'aide du processeur (120, 620), un facteur d'échelle, sur la base du signe de changement de la pression artérielle ; et
estimer (440), à l'aide du processeur (120, 620), la pression artérielle, sur la base de la valeur de la caractéristique et du facteur d'échelle,
**caractérisé par le fait que** l'étape de détection (430) du signe de changement de la pression artérielle comprend la détection du signe de changement de la pression artérielle, en fonction de la valeur de caractéristique acquise qui est supérieure à une valeur de caractéristique de référence acquise à partir du biosignal mesuré au moment de l'étalonnage, lorsqu'une condition prédéfinie pour effectuer l'étalonnage est remplie.

11. Procédé selon la revendication 10 comprend en outre la génération, à l'aide du processeur (120, 620), sur la base des informations relatives à la pression artérielle, d'un modèle d'estimation du facteur d'échelle pour chacun des signes de changement de pression artérielle positif et négatif, le modèle d'estimation du facteur d'échelle étant destiné à mettre à l'échelle une valeur de caractéristique de signal biologique à utiliser pour estimer une pression artérielle,

dans lequel le modèle d'estimation du facteur d'échelle comprend une valeur constante ou une combinaison de valeurs constantes, une première équation d'estimation reflétant les caractéristiques des utilisateurs et une seconde équation d'estimation reflétant les caractéristiques de chacun des groupes d'utilisateurs, et comprenant en outre les opérations suivantes :

classer, à l'aide du processeur (120, 620), les utilisateurs, sur la base d'un signe de changement de pression artérielle d'une valeur de caractéristique de signal biologique pour chacun des utilisateurs ;
acquérir, à l'aide du processeur, un facteur d'échelle optimal de chacun des utilisateurs, sur la base de la valeur de caractéristique de signal biologique et d'une valeur de pression artérielle réelle de chacun des utilisateurs, la valeur de caractéristique de signal biologique et la valeur de pression artérielle réelle correspondant au signe de changement de pression artérielle de chacun des utilisateurs ; et
générer, à l'aide du processeur, la première équation d'estimation reflétant les caractéristiques des utilisateurs, sur la base d'un facteur de caractéristique personnelle et du facteur d'échelle optimal de chacun des utilisateurs, ou

comprenant en outre les opérations suivantes :

classer, à l'aide du processeur (120, 620), les utilisateurs, sur la base d'un signe de changement de pression artérielle d'une valeur de caractéristique de signal biologique pour l'un des utilisateurs respectifs;
sous-classer, à l'aide du processeur, les utilisateurs classés dans les groupes, sur la base de l'un ou de toute combinaison du sexe, de l'âge, de la prise de médicaments, de la profession et de la maladie ; et générer, à l'aide du processeur, la deuxième équation d'estimation reflétant les caractéristiques de chacun des groupes.

12. Procédé selon les revendications 10 ou 11 comprend en outre la transmission, par une interface de communication, du modèle d'estimation du facteur d'échelle à un appareil d'estimation de la pression artérielle, sur la base d'une demande reçue de l'appareil d'estimation de la pression artérielle ou sur la base de la génération du modèle d'estimation du facteur d'échelle.

13. Procédé selon l'une des revendications 10 à 12, comprenant en outre le stockage, par une mémoire, de l'une ou des deux informations relatives à la pression artérielle et du modèle d'estimation du facteur d'échelle.

14. Support lisible par ordinateur comportant des instructions qui, lorsqu'elles sont exécutées par un processeur (120, 620) d'un appareil d'estimation de la pression artérielle doté d'un capteur (110, 720, 820) configuré pour mesurer (410) un biosignal d'un utilisateur, amènent le processeur (120, 620) à :

acquérir (420) une valeur de caractéristique actuelle à partir du signal biologique ;
déterminer si la valeur actuelle de la caractéristique est supérieure à une valeur de référence de la caractéristique ;
sur la base de la valeur de caractéristique actuelle déterminée comme étant supérieure à la valeur de caractéristique de référence, détecter (430) un signe de changement de la pression artérielle sur la base de la valeur de caractéristique ;
acquérir un facteur d'échelle fondé sur le signe de changement de la pression artérielle ;
mettre à l'échelle la valeur actuelle de la caractéristique à l'aide du facteur d'échelle ; et
estimer (440) la pression artérielle, sur la base de la valeur de la caractéristique actuelle mise à l'échelle, dans lequel le processeur (120, 620) est en outre configuré pour détecter le signe de changement de la pression artérielle, selon que la valeur de caractéristique acquise est supérieure à une valeur de caractéristique de référence acquise à partir du biosignal mesuré au moment de l'étalonnage, lorsqu'une condition prédéfinie pour effectuer l'étalonnage est remplie.

15. Support lisible par ordinateur selon la revendication 14, comportant des instructions pour amener le processeur (120, 620) à :

sur la base de la valeur de caractéristique actuelle déterminée comme étant supérieure à la valeur de caractéristique de référence : sélectionner un groupe auquel l'utilisateur appartient, sur la base d'informations caractéristiques de l'utilisateur ; et
acquérir le facteur d'échelle, en utilisant une équation d'estimation prédéfinie pour le groupe sélectionné et pour le cas où la valeur de caractéristique actuelle est déterminée comme étant supérieure à la valeur de caractéristique de référence ; et
sur la base de la valeur de caractéristique actuelle déterminée comme étant inférieure à la valeur de caractéristique de référence, régler le facteur d'échelle à une valeur prédéfinie pour le cas où la valeur de caractéristique actuelle est déterminée comme étant inférieure à la valeur de caractéristique de référence, ou
les instructions permettent au processeur (120, 620) de :

sélectionner un groupe auquel l'utilisateur appartient, sur la base d'informations caractéristiques de l'utilisateur ; sur la base de la valeur de la caractéristique actuelle déterminée comme étant supérieure à la valeur de la caractéristique de référence, acquérir le facteur d'échelle, en utilisant une première équation d'estimation qui est prédéfinie pour le groupe sélectionné et pour le cas où la valeur de la caractéristique actuelle est déterminée comme étant supérieure à la valeur de la caractéristique de référence ; et
sur la base de la valeur de la caractéristique actuelle déterminée comme étant inférieure à la valeur de la caractéristique de référence, acquérir le facteur d'échelle, en utilisant une deuxième équation d'estimation qui est prédéfinie pour le groupe sélectionné et pour le cas où la valeur de la caractéristique actuelle est déterminée comme étant inférieure à la valeur de la caractéristique de référence.

# FIG. 1A

# FIG. 1B

# FIG. 2A

# FIG. 2B

# FIG. 3A

# FIG. 3B

# FIG. 3C

# FIG. 3D

07:45 A.M.

○ BLOOD PRESSURE ESTIMATION

PLEASE INPUT WHETHER YOU TAKE BLOOD PRESSURE MEDICATION OR NOT

TAKE     NOT TAKE

# FIG. 3E

07:45 A.M.

BLOOD PRESSURE
ESTIMATION

PLEASE INPUT
YOUR DATE
OF BIRTH

FIG. 3F

07:45 A.M.

○ BLOOD PRESSURE
CALIBRATION

▶ BASELINE BLOOD
PRESSURE
120/75 mmHg

BLOOD PRESSURE
혈압 120/75 mmHg

# FIG. 3G

07:45 A.M.

O BLOOD PRESSURE
ESTIMATION

▶ INPUT BASELINE
BLOOD PRESSURE

SYSTOLIC [    ]

DIASTOLIC [    ]

[CONFIRM] [CANCEL]

30

32

# FIG. 4

START

```
                                    ┌─410
          ┌─────────────────────────────────┐
          │      MEASURE BIO-SIGNAL          │
          └─────────────────────────────────┘
                                    ┌─420
          ┌─────────────────────────────────┐
          │         ACQUIRE FEATURE          │
          │     VALUE FROM BIO-SIGNAL        │
          └─────────────────────────────────┘
                                    ┌─430
          ┌─────────────────────────────────┐
          │    DETECT BLOOD PRESSURE         │
          │      CHANGE SIGN AND             │
          │   ACQUIRE SCALE FACTOR           │
          └─────────────────────────────────┘
                                    ┌─440
          ┌─────────────────────────────────┐
          │   ESTIMATE BLOOD PRESSURE        │
          │   ON THE BASIS OF FEATURE        │
          │   VALUE AND SCALE FACTOR         │
          └─────────────────────────────────┘
                                    ┌─450
          ┌─────────────────────────────────┐
          │ OUTPUT RESULT OF ESTIMATION      │
          │      OF BLOOD PRESSURE           │
          └─────────────────────────────────┘
```

END

# FIG. 5A

START

$f_{cur} > f_{cal}$ ⌐511

YES:(+)  NO:(−)

SELECT GROUP ON THE BASIS OF USER CHARACTERISTIC INFORMATION ⌐513

SF = SF$_{pos}$ ⌐512

SF = $f_{neg,G}(x)$ ⌐514

END

# FIG. 5B

START

$f_{cur} > f_{cal}$ — 521

YES:(+)  NO:(-)

SELECT GROUP ON THE BASIS OF USER CHARACTERISTIC INFORMATION — 522

$SF = f_{pos,G}(x)$ — 523

$SF = SF_{neg}$ — 524

END

# FIG. 5C

START

531
SELECT GROUP ON
THE BASIS OF USER
CHARACTERISTIC
INFORMATION

532
YES:(+) $f_{cur} > f_{cal}$ NO:(-)

533
$SF = f_{pos,G}(x)$

534
$SF = f_{neg,G}(x)$

END

# FIG. 5D

START

$f_{cur} > f_{cal}$ — 541

YES:(+)  NO:(-)

SF = SF$_{pos}$ — 542

SF = SF$_{neg}$ — 543

END

FIG. 6

# FIG. 7A

# FIG. 7B

714

♥ BLOOD PRESSURE INFORMATION ♥
118/78

DATE OF MEASUREMENT: 2018-05-15 10:00
HEALTH STATUS: GOOD

714a

714b

M    I

# FIG. 8

**EP 3 636 146 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018177465 A1
- EP 0956815 A1
- US 2017181649 A1
- US 5533511 A